# DEMANDE DE BREVET EUROPEEN

(11) **EP 0 676 214 A1**
(43) Date de publication de la demande: **11.10.1995**
(21) Numéro de dépôt: 95410015.2
(22) Date de dépôt: 10.03.1995
(51) Int. Cl.: A61M 5/155

(54) **Dispositif d'injection de liquide médical**

(30) Priorité: 11.03.1994 FR 9403020
(71) Demandeur: Medex (société anonyme), F-74966 Annecy Cédex (FR)
(72) Inventeur: Lacroix, Jean-Pierre, F-74960 Annecy le Vieux (FR)
(74) Mandataire: Gasquet, Denis

(57) **Abrégé**

Dispositif destiné pour l'injection de liquide médical du type qui consiste à utiliser une poche souple (18) contenant ledit liquide (19) à injecter, à déformer la poche souple (18) par l'intermédiaire d'un liquide moteur inerte (19), mis en pression en la plaçant dans un boîtier (20) comprenant le liquide moteur inerte (22), puis à mettre en pression ledit liquide moteur, ledit boîtier étant constitué par deux demi-coques (20', 20'') mobiles l'une par rapport à l'autre entre une position d'ouverture et une position de fermeture dont les enceintes respectives (39, 40) sont fermées par une paroi de fermeture (37, 38) réalisée dans une feuille de matériau déformable élastiquement, la poche souple (18) contenant le liquide médical (19) étant disposée entre les deux parois de fermeture (37, 38), caractérisé en ce que dans la position d'ouverture, l'une des demi-coques (20') est inclinée de façon à ce que le plan général (P1) et la paroi déformable (37) forment avec le plan horizontal (H) un angle (α) compris entre 0 et 70 degrés.

## Description

La présente invention concerne un dispositif d'injection d'un liquide médical destiné au corps humain ou animal. Il est plus particulièrement relatif à un perfectionnement permettant de mettre en pression le liquide médical à injecter quand il est contenu dans une enveloppe souple.

Les récents développements de la médecine ont conduit notamment à mettre au point différents procédés d'analyse et de contrôle de l'état des patients. Parmi ces procédés il existe les analyses faites après injection d'un produit de contraste. C'est par exemple le cas pour les analyses du type artériographique qui consistent à injecter un produit iodé dans les vaisseaux, veines et artères pour opacifier l'ensemble de ceux-ci ainsi que les tissus et organes imprégnés d'opacifiant et ainsi, par radio, déterminer les anomalies éventuelles, comme les thromboses, les embolies, les compressions et autres. Il peut s'agir aussi d'utilisation de produits de contraste pour réaliser un scanner ou en angiographie traditionnelle ou numérisée, l'injection veineuse ou artérielle devant se faire sous forme de bolus à vitesses allant généralement de 0,5 à 35 ml/s. Mais il existe aussi bien d'autres types d'injection sans pour autant que ce soient des injections de liquide de contraste. C'est par exemple le cas des transfusions ou des procédés de nutrition artificielle, notamment par le sang ou par voie digestive.

On connaît déjà des injecteurs qui comprennent des seringues contenant le liquide à injecter dont la tête d'injection est reliée à une aiguille hypodermique ou intraveineuse ou à un cathéter par l'intermédiaire d'une canalisation qui comprend éventuellement une valve anti-retenue à seuil. La préparation des seringues actuelles est faite par remplissage et débullage à partir de flacons de différentes contenances telles que 60, 100, 200 millilitres, voire même plus, comme, par exemple, 500 millilitres. Devant l'exigence croissante des utilisateurs dans le domaine de l'hygiène et de la non contamination par les patients des produits utilisés, qui peuvent servir à plusieurs patients, il est donc souhaitable d'éviter au maximum les transvasements de flacon à seringue surtout si les seringues sont appelées à servir pour plusieurs patients. Le but étant d'éviter tout risque de contamination rétrograde. Le développement des maladies contagieuses, notamment du Sida, conduit les médecins à une exigence encore plus importante dans le domaine de l'hygiène et il est donc déterminant et très important de supprimer tout risque de contamination et donc de supprimer au maximum les transvasements de produits initialement nécessaires et ce dans un but principal d'hygiène et de sécurité. Les constructeurs ont donc imaginé des dispositifs dans lesquels le liquide médical est utilisé directement dans un conditionnement constitué par une poche souple munie d'un raccord. Ladite poche remplaçant ainsi la seringue traditionnelle qui est connectée à une canalisation reliée à un conduit d'injection tel qu'un cathéter ou une aiguille hypodermique, intraveineuse ou autre, est placée dans un boîtier comprenant le liquide moteur inerte mis sous pression. De tels dispositifs sont, par exemple, décrits dans la demande de brevet européen 0 343 286, dans le brevet américain 3 199 511, ainsi que dans la demande de brevet française 2 592 306. Dans tous ces dispositifs il est fait usage d'une poche souple contenant le liquide à injecter, laquelle poche est placée dans une enceinte contenant le liquide moteur qui, mis sous pression, aura pour mission de propulser le liquide contenu dans la poche. Toutefois ces dispositifs qui ne sont basés que sur des principes, n'apportent pas de véritables solutions techniques leur permettant d'être utilisés dans la pratique.

La présente invention propose donc un nouveau dispositif simple, fiable et dont l'utilisation est d'un accessible à tous.

Ainsi, il est proposé un dispositif destiné à mettre en oeuvre un procédé d'injection de liquide à usage médical du type qui consiste
- à utiliser une poche souple contenant ledit liquide médical à injecter, ladite poche étant étanche et comprenant au moins un orifice d'écoulement destiné à être connecté à une canalisation reliée à un conduit d'injection tel qu'un cathéter ou une aiguille hypodermique, intraveineuse ou autre,
- à déformer la poche souple par l'intermédiaire d'un liquide moteur inerte, mis en pression de façon à transmettre cette pression au liquide médical à injecter afin de le forcer à s'écouler dans la canalisation en la plaçant dans un boîtier comprenant le liquide moteur inerte, puis à mettre en pression ledit liquide moteur, ledit boîtier étant constitué par deux demi-coques mobiles l'une par rapport à l'autre entre une position d'ouverture permettant l'introduction de la poche souple et une position de fermeture dans laquelle les deux coques sont verrouillées l'une avec l'autre, dont les enceintes respectives sont fermées par une paroi de fermeture réalisée dans une feuille de matériau déformable élastiquement, la poche souple contenant le liquide médical étant disposée entre les deux parois de fermeture,
   et qui est caractérisé en ce que dans la position d'ouverture, l'une des demi-coques est inclinée de façon à ce que le plan général et la paroi déformable forment avec le plan horizontal un angle compris entre 0 et 70 degrés.

Selon une caractéristique complémentaire, l'angle est compris entre 30 et 50 degrés., et est avantageusement d'environ 45 degrés.

Selon une autre caractéristique complémentaire, la demi-coque inclinée est fixe tandis que l'autre demi-coque est mobile entre la position de fermeture et la position d'ouverture et inversement.

Dans un mode d'exécution préféré, la demi-coque mobile est mobile en pivotement par rapport à la demi-coque fixe, tandis que les parois déformables de fermeture sont en silicone et constituées par une membrane de faible épaisseur bordée par un bourrelet périphérique.

La disposition inclinée des coques permet, d'une part, la mise en place correcte et facile de la poche dans le boîtier et, d'autre part, l'évacuation des bulles d'air. Notons aussi que, grâce au dispositif tel qu'il est conçu selon l'invention, la pression transmise sur la poche est uniformément répartie ce qui évite tout risque de détérioration de son enveloppe.

D'autres caractéristiques et avantages de l'invention se dégageront de la description qui va suivre en regard des dessins annexés qui ne sont donnés qu'à titre d'exemples non limitatifs.

La figure 1 est une vue illustrant de façon schématique l'ensemble du dispositif d'injection.

Les figures 2 et 3 sont des vues montrant un exemple de poche déformable contenant le liquide médical, destinée à être utilisée dans le dispositif de l'invention ; la figure 2 étant une vue extérieure frontale avec arrachement partiel, la figure 3 étant une vue latérale, également avec arrachement partiel.

Les figures 4 à 7 représentent un premier mode d'exécution.

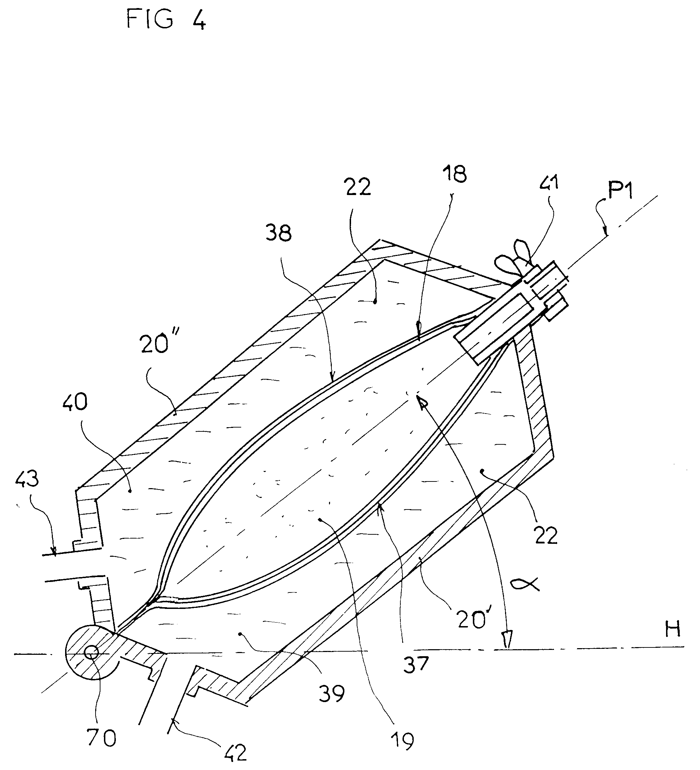

La figure 4 est une vue en coupe longitudinale du dispositif dans sa position fermée active avant injection.

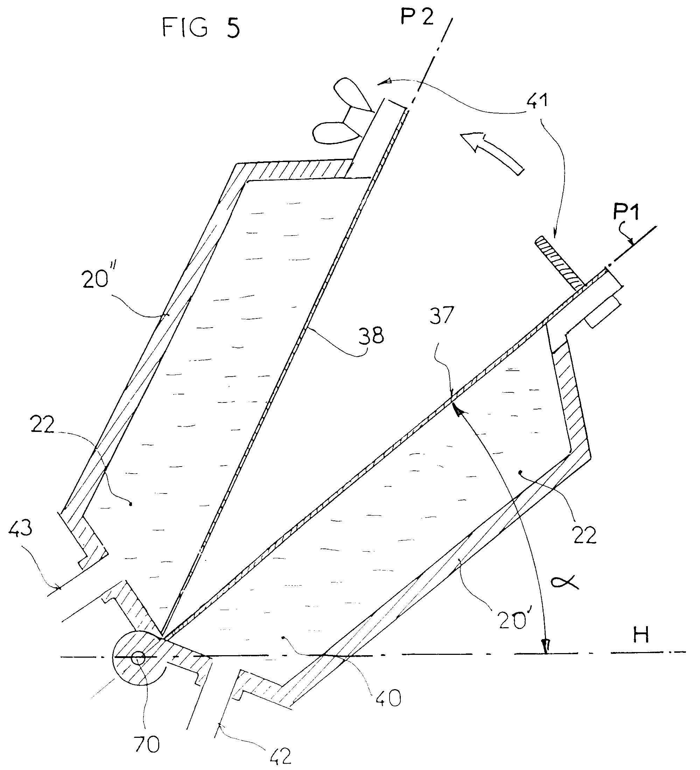

La figure 5 est une vue en coupe longitudinale du dispositif dans sa position ouverte avant l'introduction de la poche souple.

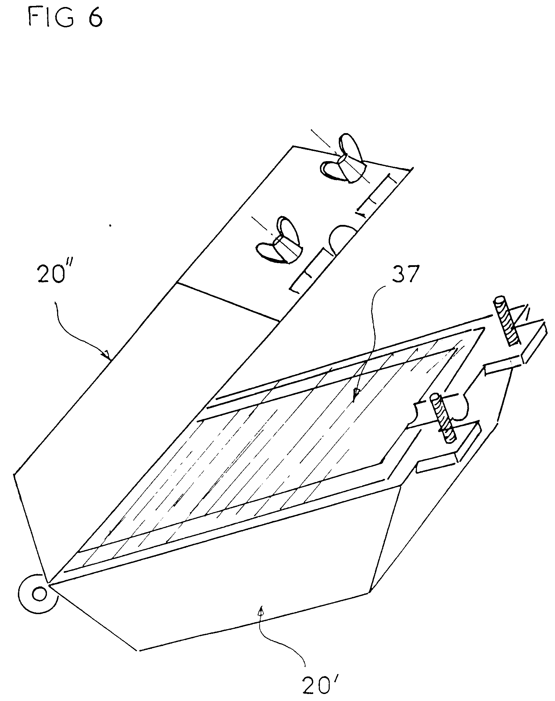

La figure 6 est une vue en perspective du dispositif dans sa position ouverte avant l'introduction de la poche souple.

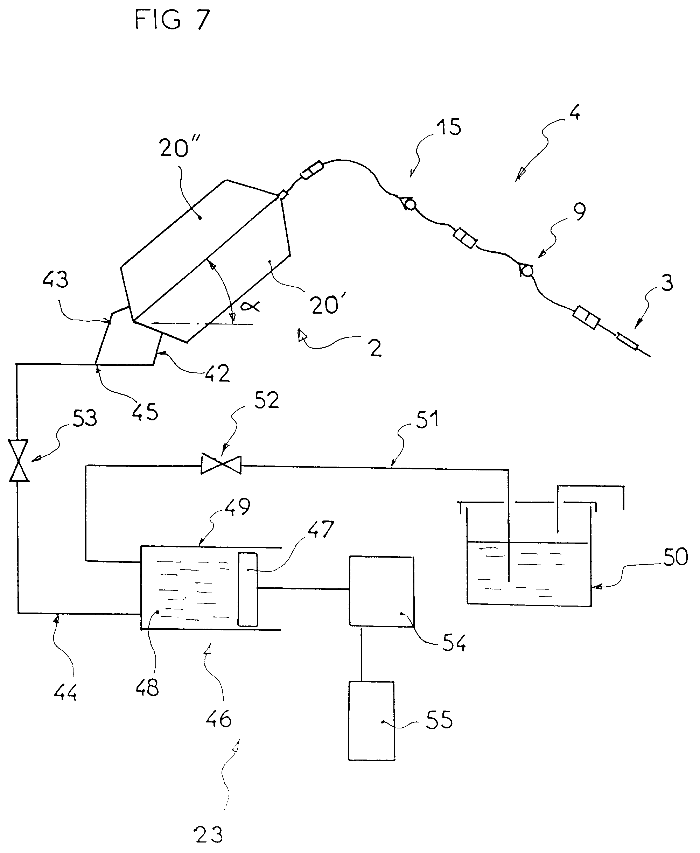

La figure 7 est un schéma d'ensemble de l'installation selon l'invention.

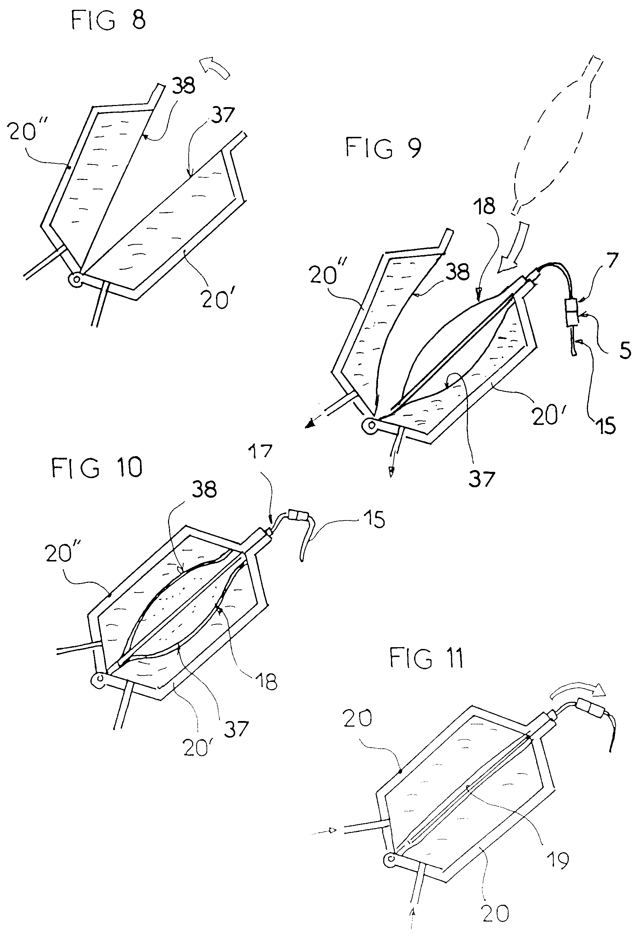

Les figures 8 à 11 sont des vues schématiques montrant les différentes étapes d'utilisation du dispositif selon l'invention.

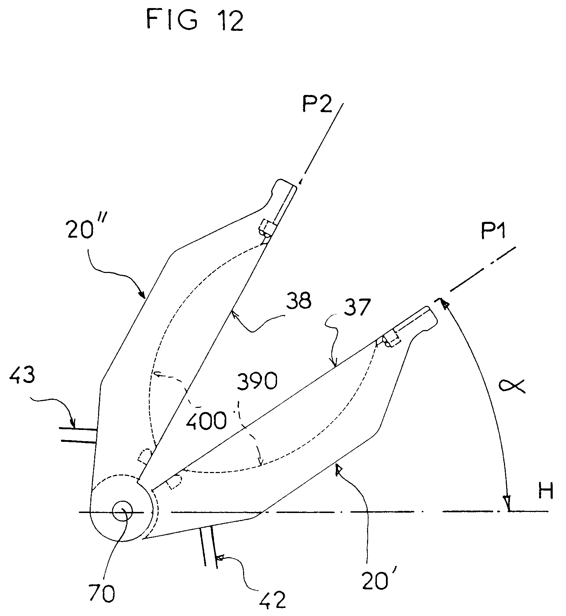

Les figures 12 à 18 représentent une variante d'exécution.

La figure 12 est une vue latérale extérieure du dispositif dans sa position ouverte.

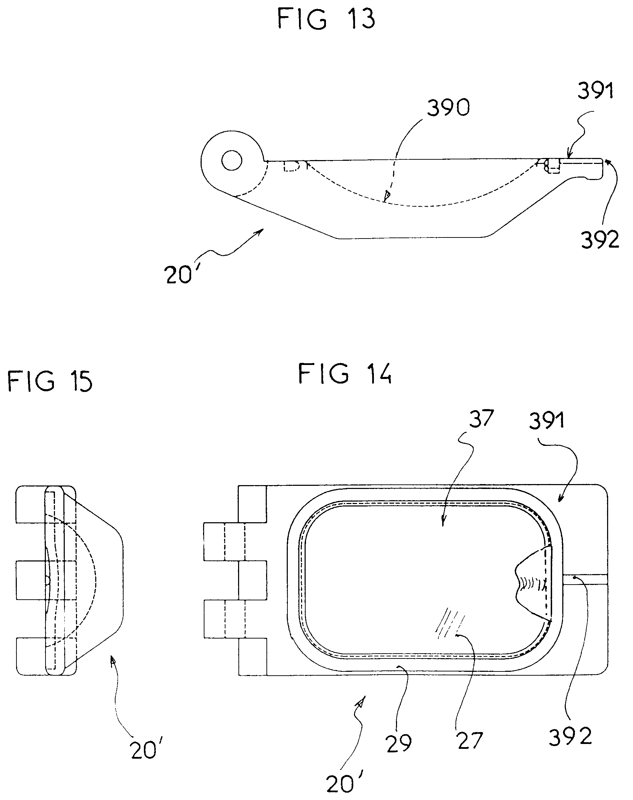

Les figures 13 à 18 sont des vues de détails des demi-coques constituant le boîtier.

Les figures 13 à 15 représentent la demi-coque inférieure.

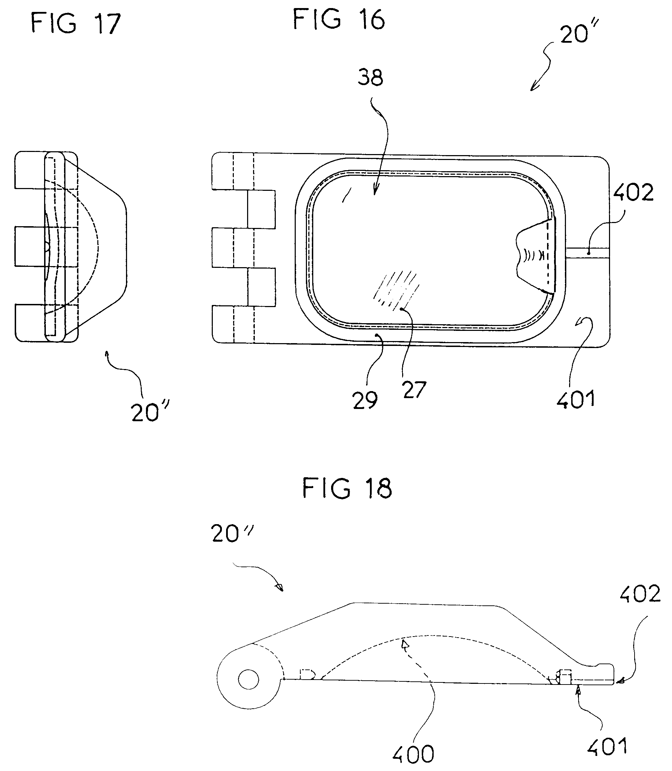

Les figures 16 à 18 représentent la demi-coque supérieure.

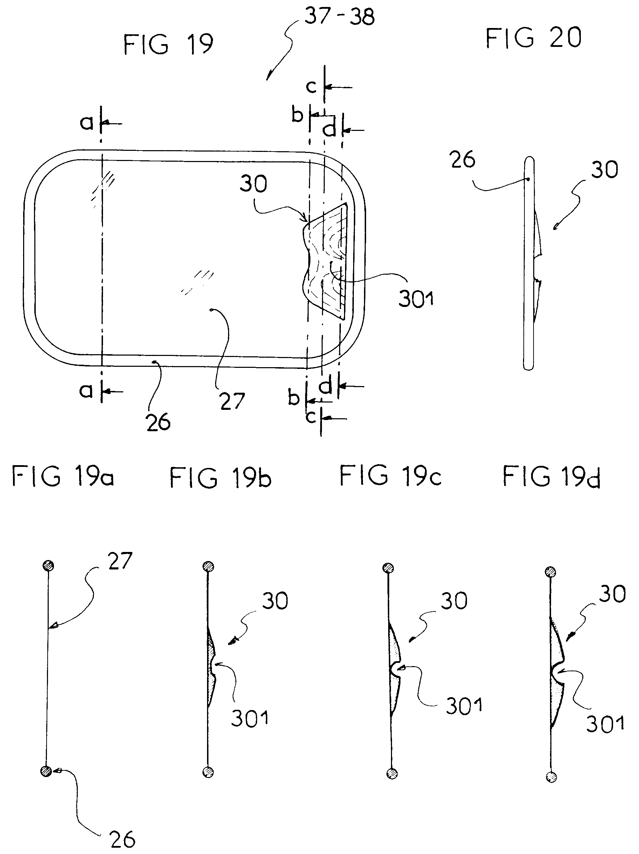

Les figures 19, 19a, 19b, 19c, 19d et 20 représentent une paroi déformable de fermeture.

La figure 19 est une vue de dessus.

La figure 19a est une vue en coupe selon a-a.

La figure 19b est une vue en coupe selon b-b.

La figure 19c est une vue en coupe selon c-c.

La figure 19d est une vue en coupe selon d-d.

La figure 20 est une vue latérale.

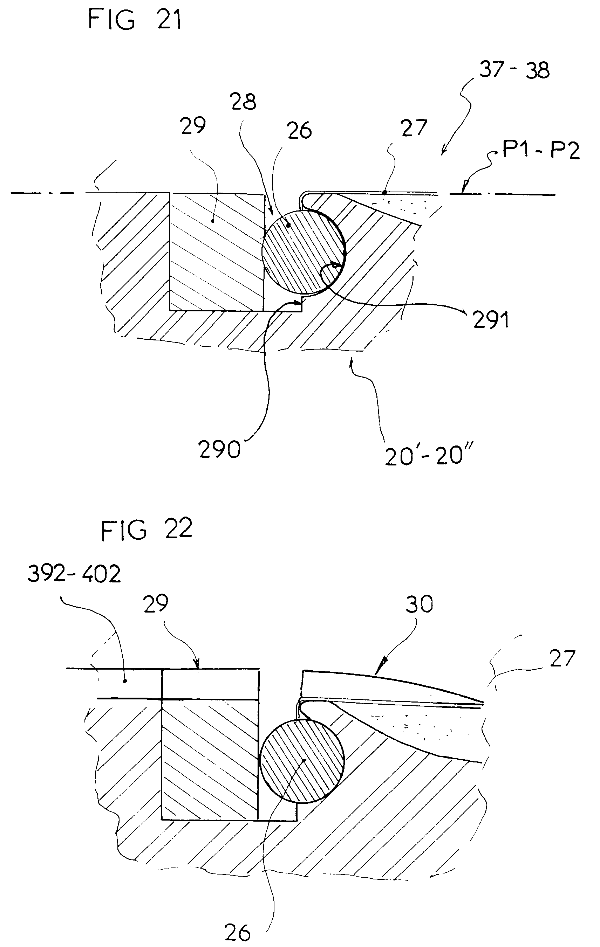

La figure 21 est une vue de détail représentant le montage de la membrane déformable de fermeture de l'enceinte sur la demi-coque.

La figure 22 est une vue similaire à la figure 21, mais selon laquelle la coupe est faite au niveau du profil en creux de passage de la canule.

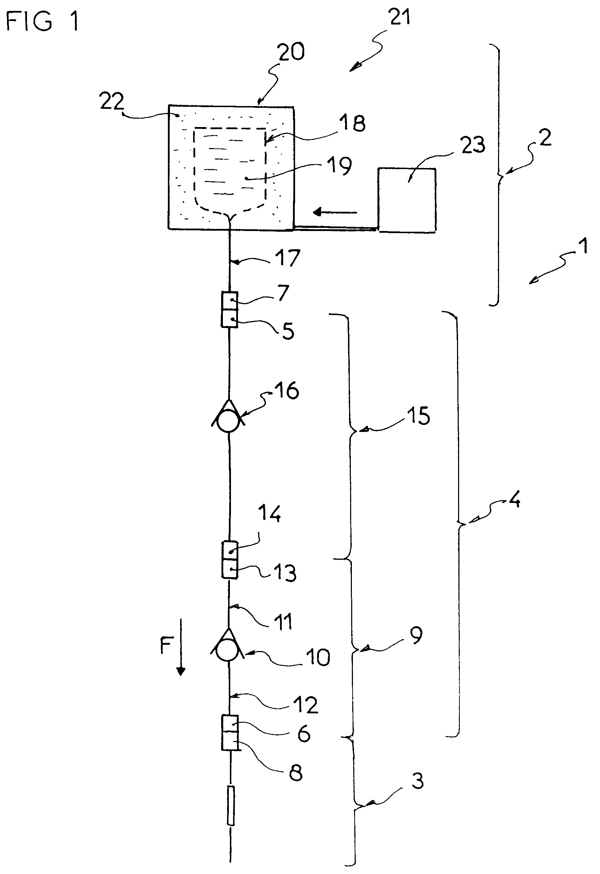

La figure 1 illustre schématiquement l'ensemble du dispositif d'injection selon l'invention qui porte la référence globale (1). Ledit dispositif comprend un injecteur proprement dit ou dispositif d'alimentation (2) relié à un conduit d'injection (3) tel qu'un cathéter, une aiguille hypodermique ou intraveineuse par l'intermédiaire d'une canalisation (4) constituée de plusieurs éléments. Ladite canalisation (4) comprend, à ses extrémités, deux raccords de branchement, un raccord amont (5) et un raccord aval (6). Le raccord amont (5) est destiné à être raccordé à un raccord d'alimentation (7) solidaire de l'injecteur, tandis que le raccord aval (6) est destiné à être raccordé au raccord amont (8) du conduit d'injection (3). Par ailleurs et avantageusement, la canalisation (4) comprend un dispositif de sécurité (9) tel que décrit dans la demande française n° 93 12590 et qui est constitué par un raccord amovible comprenant une valve anti-retour à seuil (10) comprise entre une canalisation amont (11) et une canalisation aval (12). La valve anti-retour à seuil (10) est adaptée pour autoriser le passage du liquide d'amont en aval comme représenté par la flèche "F" lorsqu'une pression déterminée du fluide en écoulement est atteinte, tandis qu'elle condamne le passage du liquide médical en sens inverse, à savoir, d'aval en amont, c'est-à-dire dans le sens contraire de celui illustré par la flèche "F". Notons que la canalisation amont (11) du dispositif de sécurité (9) comprend un raccord amont (13) destiné à être raccordé à un raccord aval (14) d'une portion amont de la canalisation (4) qui comprend par ailleurs une autre valve anti-retour à seuil (16), tandis que la canalisation aval (12) comprend le raccord aval (6) précédent décrit.

Ajoutons que la canalisation (4) est connectée à l'injecteur (2) par raccordement du raccord amont (5) de ladite canalisation au raccord d'alimentation (7). Selon l'une des caractéristiques de l'invention, ledit raccord (7) est directement fixé à l'extrémité du conduit d'alimentation (17) d'une poche souple (18) contenant le liquide médical (19) à injecter. Ladite poche souple (18) étant disposée dans le boîtier de mise en pression (20) du dispositif de mise en pression (21). Selon l'invention, la mise en pression de la poche souple est réalisée par un liquide moteur inerte enveloppant ladite poche. Ledit liquide moteur inerte (22) étant contenu dans le boîtier (20), et sa mise en pression étant réalisée par des moyens de mise en pression (23) tels qu'une pompe haute pression ou un vérin comprenant un piston coulissant actionné par un moteur électrique dont l'avance est contrôlée, ou tout autre dispositif motorisé voire même manuel.

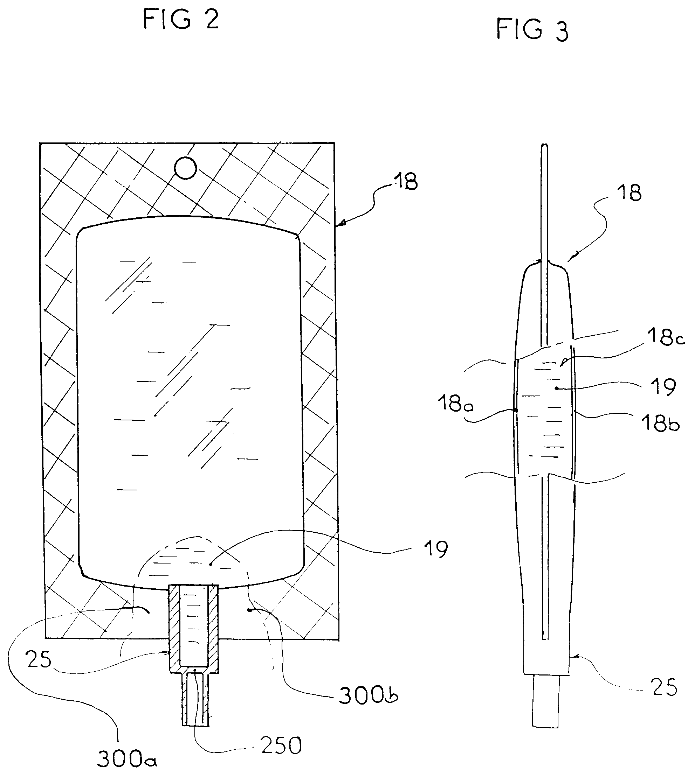

Les figures 2 et 3 illustrent schématiquement une poche (18) telle qu'utilisée dans le procédé selon l'invention et contenant le liquide médical (19) à injecter. Ladite poche (18) est constituée par l'assemblage de deux feuilles en matériau plastique souple, une première feuille (18a) et une deuxième feuille identique (18b) associées périphériquement par collage ou soudage de façon à constituer un volume interne (18c) destiné à contenir le liquide médical (19). Par ailleurs, la partie inférieure de la poche comprend un tube d'écoulement (25) obturé provisoirement par une membrane (250) que l'on perce lors du branchement dudit tube sur le conduit d'alimentation (17). La poche ainsi réalisée et contenant le liquide est molle et déformable et est, selon l'invention, mise en place dans le liquide moteur (22) d'un boîtier (20), ledit liquide enveloppant la totalité de la poche, étant mise en pression pour déformer ladite poche de façon homogène et périphérique, afin de forcer le liquide médical à s'écouler dans la canalisation (4).

Un premier mode de réalisation du dispositif de mise en pression est représenté schématiquement aux figures 4 à 7.

Selon ce premier mode réalisation, le boîtier de mise en pression (20) est constitué par deux demi-coques (20', 20'') articulées l'une par rapport à l'autre autour d'un axe (70). Chacune des coques comporte une paroi de fermeture (37, 38) pour former deux demi-enceintes (39, 40) comprenant le liquide moteur inerte (22). Les demi-coques (20', 20'') sont réalisées, par exemple, en aluminium ou en matériau composite avec de la fibre de verre ou de la fibre de carbone, tandis que les parois de fermeture (37, 38) sont réalisées par une feuille de matériau déformable élastiquement telle qu'en caoutchouc naturel ou synthétique, mais avantageusement en silicone.

L'une des demi-coques (20') est, par exemple, fixe tandis que l'autre des demi-coques (20'') est mobile par rapport à la première. Ainsi le dispositif peut avoir une position ouverte tel que cela est représenté à la figure 5 et avoir une position fermée telle qu'illustrée à la figure 4. Ladite position ouverte permet la mise en place de la poche comme nous l'expliquerons plus loin, tandis que la position fermée permet l'injection du liquide médical par mise en pression du liquide moteur (19) contenu dans les demi-enceintes (39, 40). Notons de plus que les deux demi-coques (20', 20'') sont maintenues dans leur position de fermeture par des moyens de fermeture (41) tels qu'une vis, un boulon ou tout autre dispositif ou, par exemple, un système de verrouillage automatique. On pourrait notamment prévoir un système de fermeture tel que, dès son déverrouillage, cela provoque automatiquement l'ouverture de la demi-coque mobile.

Chacune des enceintes (39, 40) est alimentée en liquide moteur (22) par un conduit d'arrivée respectivement (42, 43). Chacun de ces conduits étant connecté à une tubulure (44) par l'intermédiaire d'une dérivation (45), ladite tubulure comprenant une vanne principale électrique (53) est reliée à un vérin (46) dont le piston (47) est commandé en déplacement par un moteur électrique (54) dont l'avance est contrôlée par un automatisme (55). Grâce à la dérivation les deux enceintes (39, 40) se trouvent être en communication l'une avec l'autre de façon à obtenir la même pression dans celles-ci.

Par ailleurs, la chambre (48) du cylindre (49) du vérin (46) est reliée à un réservoir supplémentaire de liquide moteur (50) par un conduit secondaire (51) comprenant une vanne secondaire électrique (52). La poche déformable (18) contenant le liquide médical à injecter (19) est destinée à être placée entre les deux parois de fermeture (37, 38).

Ainsi, dans une phase préliminaire d'utilisation du dispositif pendant laquelle est faite la remise à zéro, il est prévu de fermer les deux demi-coques sans y avoir mis en place une poche de liquide à injecter, les deux vannes (52, 53) étant ouvertes, le piston (47) étant ensuite reculé, la vanne (52) étant alors fermée, l'affichage du zéro étant alors fait par avance du piston.

Les coques (20', 20'') sont ensuite ouvertes comme cela est illustré à la figure 8. La vanne (52) étant fermée, tandis que la vanne (53) est ouverte, le piston (47) du vérin (46) est alors reculé pour créer une dépression dans les enceintes (39, 40) les vidant ainsi de leur liquide comme cela est illustré à la figure 9. On peut noter que dans cette position les parois déformables (37, 38) se creusent pour créer ainsi un espace destiné à recevoir la poche souple (18). La dépression créée étant suffisante, pour que la poche trouve sa place dans l'espace librement et sans contrainte. La poche (18) est alors introduite entre les deux coques. Puis la coque mobile (20'') est refermée sur la coque fixe (20') et les deux coques sont verrouillées l'une avec l'autre comme cela est illustré à la figure 10. La vanne électrique (52) est alors fermée tandis que la vanne principale (53) est ouverte. Le déplacement du vérin (47) est ensuite commandé pour mettre en pression le liquide moteur des deux enceintes et chasser ainsi le liquide médical de façon à réaliser l'injection comme cela est représenté schématiquement figure 11.

Selon l'invention, le plan géneral (P1) de la paroi déformable (37) de la demi-coque fixe (20') est, dans la position d'ouverture inclinée pour former avec le plan horizontal (H) un angle aigu (α), compris entre 0 et 70 degrés et qui avantageusement est compris entre 30 et 50 degrés et est, par exemple, d'environ 45 degrés. Avec une telle disposition, et comme cela apparaît plus précisément à la figure 9, il est possible à l'utilisateur du dispositif de poser la poche souple (18) sur la paroi déformable (37) sans précaution particulière car ladite poche restera correctement en place sans bouger pendant la fermeture du boîtier.

La figure 12 illustre un mode d'exécution préféré, selon lequel les éléments identiques à ceux du mode de réalisation de principe des figures 4 à 11 précédemment décrits seront repris et il suffit au lecteur pour la compréhension du mode préféré de se reporter à la description du mode des figures 4 à 11. Ajoutons toutefois les commentaires ci-après.

Dans cette réalisation, chacune des demi-coques (20', 20'') présente une conformation profilée dont les formes extérieures lui donnent un aspect ornemental plus séduisant. Par ailleurs, les deux enceintes (39, 40) qui comprennent le liquide moteur (22) sont limitées chacune par une paroi concave courbe respectivement (390, 400). Notons aussi que la zone supérieure du plan de joint (391, 401) de chacune des demi-coques comprend, bien entendu, un profil en creux demi-cylindrique (392, 402) permettant le passage du tube d'écoulement (25) de la poche.

La paroi déformable (37, 38) de chacune des demi-coques a la forme générale d'un rectangle à angles arrondis. Elle est avantageusement en silicone et comprend un bourrelet périphérique (26) tel qu'illustré aux figures 19, 19a, 19b, 19c, 19d et 20. Ledit bourrelet périphérique (26) ayant la forme d'un profil cylindrique est moulé avec la membrane (27) de la paroi déformable (37, 38). Il constitue un moyen de retenue pour la membrane (27) par engagement dans un profil en creux périphérique (28) réalisé dans chacune des demi-coques, profil en creux ouvert sur le plan de joint et qui reçoit aussi une pièce de verrouillage (29). Ledit profil en creux a une section transversale de forme générale carrée et sa paroi interne (290) comprend une gorge (291) dont le profil est demi-cylindrique pour recevoir le joint périphérique (26) de la membrane, comme cela apparaît plus visiblement à la figure (21).

Chacune des membranes constituant la paroi déformable de fermeture (37, 38) comprend en plus du bourrelet périphérique (26) une saillie centrale supérieure (30), comme cela apparaît aux figures 19, 19a, 19b, 19c, 19d et 20.

La membrane est placée de façon à ce que la saillie (30) soit disposée hors de l'enceinte du côté de la poche et a une forme de volume évolutif, de façon à s'adapter aux zones (300a, 300b) de la poche (voir figure 2), ladite saillie comprenant un canal central (301) dans lequel prend place la tubulure (25) de la poche (18).

Il va de soi que l'on ne sortira pas du cadre de l'invention quel que soit les types de moyens de mise en pression utilisés car ceux-ci peuvent être, par exemple, aussi bien motorisés que manuels.

De même, la gestion des différentes vannes éventuelles ou organes destinés à gérer les débits, peut être automatique, semi-automatique ou manuelle. Notons qu'avantageusement les variations de volume et de débit sont constamment contrôlées afin d'assurer une injection précise et sûre.

On pourrait aussi prévoir un système de sécurité qui empêche toute injection dès que le boîtier est déverrouillé. Par ailleurs, il va de soi que le dispositif comprend un support qui peut être mobile ou fixe et qui peut être en appui sur le sol ou suspendu sur un portique ou encore fixé au plafond ou à un mur. Notons aussi que l'une des coques peut comprendre les organes de commande et de visualisation.

Bien entendu, l'invention n'est pas limitée aux modes de réalisation décrits et représentés à titre d'exemples, mais elle comprend aussi tous les équivalents techniques ainsi que leurs combinaisons.

## Revendications

1. Dispositif destiné à mettre en oeuvre un procédé d'injection de liquide à usage médical du type qui consiste
- à utiliser une poche souple (18) contenant ledit liquide médical (19) à injecter, ladite poche étant étanche et comprenant au moins un orifice d'écoulement destiné à être connecté à une canalisation (4) reliée à un conduit d'injection (3) tel qu'un cathéter ou une aiguille hypodermique, intraveineuse ou autre,
- à déformer la poche souple (18) par l'intermédiaire d'un liquide moteur inerte (19), mis en pression de façon à transmettre cette pression au liquide médical à injecter afin de le forcer à s'écouler dans la canalisation (4) en la plaçant dans un boîtier (20) comprenant le liquide moteur inerte (22), puis à mettre en pression ledit liquide moteur, ledit boîtier étant constitué par deux demi-coques (20', 20'') mobiles l'une par rapport à l'autre entre une position d'ouverture permettant l'introduction de la poche souple (18) et une position de fermeture dans laquelle les deux coques sont verrouillées l'une avec l'autre, dont les enceintes respectives (39, 40) sont fermées par une paroi de fermeture (37, 38) réalisée dans une feuille de matériau déformable élastiquement, la poche souple (18) contenant le liquide médical (19) étant disposée entre les deux parois de fermeture (37, 38),
caractérisé en ce que dans la position d'ouverture, l'une des demi-coques (20') est inclinée de façon à ce que le plan général (P1) et la paroi déformable (37) forment avec le plan horizontal (H) un angle (a) compris entre 0 et 70 degrés.

2. Dispositif d'injection selon la revendication 1, caractérisé en ce que l'angle (α) est compris entre 30 et 50 degrés.

3. Dispositif selon la revendication 2, caractérisé en ce que l'angle (α) est égal à environ 45 degrés.

4. Dispositif selon l'une quelconque des revendications précédentes, caractérisé en ce que la demi-coque inclinée (20') est fixe tandis que l'autre demi-coque est mobile entre la position de fermeture et la position d'ouverture et inversement.

5. Dispositif selon la revendication 4, caractérisé en ce que la demi-coque mobile (20'') est mobile en pivotement par rapport à la demi-coque fixe (20').

6. Dispositif selon l'une quelconque des revendications précédentes, caractérisé en ce que l'enceinte du boîtier (20', 20'') est reliée à un organe de mise en pression (34, 46) par l'intermédiaire d'au moins un conduit d'arrivée de liquide moteur (32, 42, 43, 44).

7. Dispositif selon l'une quelconque des revendications précédentes, caractérisé en ce que les parois déformables de fermeture (37, 38) sont en silicone.

8. Dispositif selon la revendication 7, caractérisé en ce que chacune des parois déformables de fermeture (37, 38) est constituée par une membrane de faible épaisseur (27) bordée par un bourrelet périphérique (26).

9. Dispositif selon la revendication 8, caractérisé en ce que la membrane (27) de la paroi déformable (37, 38) comprend une saillie centrale (30) comprenant un canal central (301).

10. Dispositif selon l'une quelconque des revendications précédentes, caractérisé en ce que chacune des enceintes (39, 40) est alimentée en liquide moteur (22) par l'intermédiaire d'un conduit d'arrivée respectivement (42, 43) relié à une tubulure d'arrivée (44) reliée à la chambre (48) d'un vérin (46) dont le piston (47) est commandé en déplacement par un moteur électrique (54) contrôlé par un automatisme (55).

11. Dispositif selon la revendication 10, caractérisé en ce que la tubulure d'arrivée (44) comprend une vanne électrique (53), tandis que la chambre (48) du vérin est reliée à un réservoir (50) par l'intermédiaire d'un conduit secondaire (51) comprenant une vanne électrique (52).
